(19) Europäisches Patentamt
European Patent Office
Office européen des brevets

(11) **EP 2 750 732 B1**

(12) **EUROPÄISCHE PATENTSCHRIFT**

(45) Veröffentlichungstag und Bekanntmachung des Hinweises auf die Patenterteilung:
**09.10.2019 Patentblatt 2019/41**

(21) Anmeldenummer: **12755797.3**

(22) Anmeldetag: **29.08.2012**

(51) Int Cl.:
***A61M 1/36*** *(2006.01)*

(86) Internationale Anmeldenummer:
**PCT/EP2012/003628**

(87) Internationale Veröffentlichungsnummer:
**WO 2013/029786 (07.03.2013 Gazette 2013/10)**

(54) **BLUTBEHANDLUNGSVORRICHTUNG**

BLOOD TREATMENT DEVICE

DISPOSITIF DE TRAITEMENT DE SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.09.2011 DE 102011112221**
**02.09.2011 US 201161530554 P**

(43) Veröffentlichungstag der Anmeldung:
**09.07.2014 Patentblatt 2014/28**

(73) Patentinhaber: **Fresenius Medical Care Deutschland GmbH**
**61352 Bad Homburg (DE)**

(72) Erfinder:
• **WIKTOR, Christoph**
**63571 Gelnhausen (DE)**
• **PETERS, Arne**
**61352 Bad Homburg (DE)**
• **HEIDE, Alexander**
**65817 Eppstein (DE)**

(74) Vertreter: **Laufhütte, Dieter et al**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
WO-A1-2008/125894 WO-A1-2011/063906
DE-A1-102007 007 198 DE-A1-102009 018 664
US-A- 4 898 576 US-A- 5 609 576

**Beschreibung**

Die vorliegende Erfindung betrifft eine Blutbehandlungsvorrichtung mit wenigstens einer Impellerpumpe sowie ein Verfahren zur Inbetriebnahme einer Blutbehandlungsvorrichtung.

Zentrifugalpumpen bzw. Impellerpumpen sind Druckquellen und finden im Bereich der Blutförderung Anwendung. Derartige Zentrifugalpumpen bzw. Impellerpumpen können als Alternative zu Verdrängerpumpen, insbesondere Schlauchrollenpumpen eingesetzt werden.

Zentrifugalpumpen bzw. Impellerpumpen erzeugen im Gegensatz zu Flussquellen wie beispielsweise Schlauchrollenpumpen keinen von der Drehzahl abhängigen Durchfluss, sondern einen von der Drehzahl abhängigen Differenzdruck. Der Durchfluss von Zentrifugalpumpen stellt sich im Wesentlichen durch den Flusswiderstand des Kreislaufs und die Viskosität der Flüssigkeit ein.

Der Flusswiderstand spielt in extrakorporalen Kreisläufen eine große Rolle: Ein veränderter Widerstand z. B. eines Dialysefilters in einem extrakorporalen Blutkreislauf zur Blutbehandlung deutet beispielsweise auf die Gerinnung von Blut hin. Sogenanntes Kinking, Clotting oder Stenosen können zu einer starken Veränderung des Widerstands der Leitungen führen. Das Ansaugen der arteriellen Nadel oder des Katheters an ein Gefäß des Patienten führt zu einem starken Anstieg des Flusswiderstands im Patientenzugang. Alle vorgenannten Ereignisse können relativ häufig während extrakorporaler Blutbehandlungen unter ungünstigen Voraussetzungen eintreten und können ferner potentiell zu einer Schädigung des Blutes führen. Vor diesem Hintergrund ist es wichtig, diese Vorgänge möglichst frühzeitig zu detektieren und Gegenmaßnahmen einzuleiten.

Aus der DE 10 2007 007 198 A1 ist bereits ein Verfahren und eine Vorrichtung zur Überwachung und Optimierung eines durch eine Pumpe bewirkten Blutkreislaufs bekannt. Dabei wird zur automatischen Regelung von Blutpumpen durch periodische Drehzahlinterventionen und die dabei auftretenden Flussänderungen über eine gebildete differentielle Größe und einen Regelalgorithmus eine Optimierung des Blutflusses erreicht. Zusätzlich kann die Lage von möglichen Flusswiderständen auf venöser oder arterieller Seite ermittelt werden.

Des Weiteren sind im Zusammenhang mit der Blutbehandlung Kassettensysteme bekannt, bei denen zumindest ein Teil des extrakorporalen Blutkreislaufs in einer disposablen Kassette zusammengefasst ist. Derartige Kassettensysteme werden beispielsweise bereits durch die DE 10 2009 024 465 A1 sowie die DE 10 2009 018 664 A1 offenbart.

Aus der DE 10 2007 007 198 A1 ist ein Verfahren zum Betreiben von Blutpumpen innerhalb eines intra- oder extrakorporalen Blutkreislaufs mit nachfolgenden Verfahrensschritten bekannt: a) Ermittlung eines Schwellenwerts T, der ein Teil eines Mittelwerts aus gemessenen Kenngrößen DFSR ("Differential Flow Speed Ratio") ist, wobei die einzelnen DFSR-Kenngrößen aus dem Quotienten einer differenziellen Blutförderleistung zu einer dazu gemessenen differenziellen Blutpumpendrehzahl errechnet werden, wobei schrittweise die Blutpumpendrehzahl bis 3/4 des im Kreislauf benötigten Blutflusses erhöht wird und für jeden Veränderungsschritt eine DFSR-Kenngröße errechnet wird; b) Reduzierung der Blutpumpendrehzahl und schrittweise Berechnung der dazugehörigen DFSR-Kenngrößen; c) Vergleich der DFSR-Kenngrößen von Verfahrensschritt b) mit dem Schwellenwert T aus Verfahrensschritt a); d) Veränderung der Blutpumpendrehzahl je nachdem ob die DFSR-Kenngröße nach Verfahrensschritt b) größer oder kleiner als der Schwellenwert T ist. Weiterhin ist dort angegeben, dass Rückflusshindernisse den Flusswiderstand auf der Einlassseite der Pumpe erhöhen, was die Pumpenvorlast reduziert und den Pumpfluss negativ beeinflusst. Im Falle von Clotting eines Oxygenators ist der Widerstand gleichfalls erhöht, aber dieser zusätzliche Widerstand tritt dann hinter der Pumpe auf. Beide Fälle beeinflussen DFSR, aber zur Unterscheidung zwischen Clotting des Oxygenators und Behinderung des venösen Rückflusses zur Pumpe kann die zusätzliche Messung des Druckabfalls über den Oxygenator (Druck am Einlass - Druck am Auslass) die benötigte Information liefern. Wenn DFSR abfällt, zeigt die Höhe des Differenzdrucks am Oxygenator die Ursache des gestiegenen Widerstands an, d. h. ob sie vor oder nach der Pumpe liegt. Falls der Differenzdruck gering ist, liegt eine Behinderung des venösen Rückflusses vor, bei hohem Differenzdruck ist Clotting des Oxygenators eingetreten.

Wünschenswert wäre es angesichts des vorgenannten Hintergrunds, eine einfache und zuverlässige Flusswiderstandsbestimmung bei Blutbehandlungsvorrichtungen mit extrakorporalen Kreisläufen, die über Zentrifugalpumpen bzw. Impellerpumpen verfügen, bereitstellen zu können.

Es ist daher die Aufgabe der vorliegenden Erfindung, eine Blutbehandlungsvorrichtung sowie ein Verfahren zur Inbetriebnahme einer Blutbehandlungsvorrichtung der eingangs genannten Art in vorteilhafter Weise weiterzubilden.

Diese Aufgabe wird erfindungsgemäß gelöst durch eine Blutbehandlungsvorrichtung mit den Merkmalen des Anspruchs 1.

Gemäß der vorliegenden Erfindung ist vorgesehen, dass eine Blutbehandlungsvorrichtung wenigstens einen extrakorporalen Blutkreislauf mit einem Filterelement sowie wenigstens ein Zentrifugalpumpmittel aufweist, wobei ferner ein Überwachungsmittel vorgesehen ist, mittels dessen der Flusswiderstand des extrakorporalen Kreislaufs bei Inbetriebnahme des extrakorporalen Kreislaufs in einem initialen Test bei kurzgeschlossenen Patientenleitungen ermittelbar ist, wobei der Flusswiderstand zumindest teilweise anhand des Flusses in wenigstens einem Abschnitt des extrakorporalen Blutkreislaufs und der Drehzahl des Zentrifugalpumpmittels ermittelbar ist und wobei der extrakorporale Blutkreislauf zumindest teilweise durch eine disposable Kassette ausgebildet ist.

Bei der Blutbehandlungsvorrichtung kann es sich beispielsweise um eine Hämodialysemaschine zum Einsatz für die Hämodialyse, Hämodiafiltration, Hämofiltration usw. handeln.

Die erfindungsgemäße Blutbehandlungsvorrichtung erlaubt es insbesondere, ein direktes Maß des Flusswiderstands eines Kreislaufs ermitteln zu können. Darüber hinaus ist es möglich, einen initialen Test von Bestandteilen des extrakorporalen Blutkreislaufs durchführen zu können. Bei diesen Bestandteilen kann es sich insbesondere um Einwegartikel handeln.

Darüber hinaus ist es möglich, beispielsweise Überwachungsmittel des extrakorporalen Blutkreislaufes wie beispielsweise Flusssensoren einem initialen, funktionalen Test unterziehen zu können.

Darüber hinaus ist es möglich, eine Flusswiderstandsmessung unabhängig von der Viskosität des geförderten Mediums im extrakorporalen Blutkreislauf durchführen zu können, was beispielsweise bei einer Druckmessung nicht der Fall ist. Denn bei Flusswiderstandsmessungen basierend auf Druckmessungen ist es erforderlich, die Viskosität des geförderten Mediums, also beispielsweise die Viskosität des Blutes ermitteln zu müssen.

Ferner ist es möglich, eine kontinuierliche Messung während der Blutflussregelung ohne einen zusätzlichen Test durchführen zu können.

Ferner ist es sehr vorteilhaft möglich, nunmehr den Zusammenhang von Drehzahl und Fluss nutzen zu können, um die Viskosität des geförderten Mediums, beispielsweise des Blutes zu bestimmen.

Erfindungsgemäß ist weiterhin vorgesehen, dass mittels des Überwachungsmittels in einem ersten Schritt der Fluss in dem wenigstens einen Abschnitt des extrakorporalen Blutkreislaufs bei einer ersten Drehzahl ermittelbar ist und in einem zweiten Schritt der Fluss in dem wenigstens einen Abschnitt des extrakorporalen Blutkreislaufs bei einer zweiten Drehzahl ermittelbar ist, wobei anhand dieser Werte als Maß für den Flusswiderstand das Verhältnis der Differenzwerte errechenbar ist. Das Maß für den Flusswiderstand ist das Verhältnis der Differenzwerte des Flusses $Q1$ in dem wenigstens einen Abschnitt des extrakorporalen Blutkreislaufs bei einer ersten Drehzahl $n1$ und dem Fluss $Q2$ in dem wenigstens einen Abschnitt des extrakorporalen Blutkreislaufs bei einer zweiten Drehzahl $n2$, also der Wert $dQ/dn$. Dabei wird mittels des Überwachungsmittels ein fehlerhafter Zustand des extrakorporalen Blutkreislaufs erkannt, wenn das Maß für den Flusswiderstand einen vordefinierten Bereich verlässt und/oder einen vordefinierten Schwellwert übersteigt oder unterschreitet. Dabei wird die Behandlung freigegeben, wenn das Maß für den Flusswiderstand $(dQ/dn)$ innerhalb vorgegebener Grenzwerte liegt, und, falls dies nicht der Fall ist, eine Fehlermeldung ausgegeben.

Des Weiteren kann vorgesehen sein, dass mittels des Überwachungsmittels bei Erkennung eines fehlerhaften Zustandes wenigstens eine Gegenmaßnahme einleitbar ist.

Es ist ferner denkbar, dass die Gegenmaßnahme das Auslösen eines Alarmes und/oder die Veränderung der Drehzahl des Zentrifugalpumpmittels und/oder die Überführung der Blutbehandlungsvorrichtung in einen sicheren Zustand umfasst.

Es ist möglich, dass wenigstens ein Anzeigemittel vorgesehen ist, mittels dessen das Maß für den Flusswiderstand darstellbar und/oder anzeigbar ist. Dadurch ergibt sich der Vorteil, eine leicht verständliche nicht technische Darstellung des Flusswiderstandes beispielsweise auf einem User Interface wie z. B. einem Display der Blutbehandlungsvorrichtung anzeigen zu können.

Darüber hinaus kann vorgesehen sein, dass im extrakorporalen Blutkreislauf wenigstens ein Druckerfassungsmittel, insbesondere wenigstens ein Drucksensor, vorgesehen ist, mittels dessen der Druck in wenigstens einem Abschnitt des extrakorporalen Blutkreislaufs ermittelbar ist.

Außerdem ist denkbar, dass mittels des Überwachungsmittels anhand des mittels des Druckerfassungsmittels ermittelten Druckes eine Stelle im extrakorporalen Blutkreislauf mit einem erhöhten Flusswiderstand ermittelbar ist.

Denkbar ist, dass die Kassette wenigstens einen Schlauchabschnitt und/oder wenigstens eine Tropfkammer und/oder wenigstens ein Ventil und/oder wenigstens einen Filter und/oder das Filterelement aufweist.

Des Weiteren betrifft die vorliegende Erfindung ein Verfahren zur Inbetriebnahme einer Blutbehandlungsvorrichtung mit den Merkmalen des Anspruchs 6.

Danach ist vorgesehen, dass bei einem Verfahren zur Inbetriebnahme einer Blutbehandlungsvorrichtung, mit wenigstens einem extrakorporalen Blutkreislauf mit einem Filterelement sowie mit wenigstens einem Zentrifugalpumpmittel, wobei ferner ein Überwachungsmittel vorgesehen ist, mittels dessen der Flusswiderstand des extrakorporalen Kreislaufs bei Inbetriebnahme des extrakorporalen Kreislaufs in einem initialen Test bei kurzgeschlossenen Patientenleitungen ermittelt, der Flusswiderstand zumindest teilweise anhand des Flusses in wenigstens einem Abschnitt des extrakorporalen Blutkreislaufs und der Drehzahl des Zentrifugalpumpmittels ermittelt wird.

Weiterhin ist vorgesehen, dass mittels des Überwachungsmittels in einem ersten Schritt der Fluss in dem wenigstens einen Abschnitt des extrakorporalen Blutkreislaufs bei einer ersten Drehzahl ermittelt wird und in einem zweiten Schritt der Fluss in dem wenigstens einen Abschnitt des extrakorporalen Blutkreislaufs bei einer zweiten Drehzahl ermittelt wird, wobei anhand dieser Werte als Maß für den Flusswiderstand das Verhältnis der Differenzwerte errechnet wird. Dabei wird mittels des Überwachungsmittels ein fehlerhafter Zustand des extrakorporalen Blutkreislaufs erkannt, wenn das Maß für den Flusswiderstand einen vordefinierten Bereich verlässt und/oder einen vordefinierten Schwellwert übersteigt oder unterschreitet, wobei die Behandlung freigegeben wird, wenn das Maß für den Flusswiderstand (dQ/dn) innerhalb vorgegebener Grenzwerte liegt, und, falls dies nicht der Fall ist, eine Fehlermeldung ausgegeben wird.

Außerdem kann vorgesehen sein, dass mittels des Überwachungsmittels bei Erkennung eines fehlerhaften Zustandes wenigstens eine Gegenmaßnahme eingeleitet wird.

Es ist ferner möglich, dass die Gegenmaßnahme das Auslösen eines Alarmes und/oder die Veränderung der Drehzahl des Zentrifugalpumpmittels und/oder die Überführung der Blutbehandlungsvorrichtung in einen sicheren Zustand umfasst.

Darüber hinaus kann vorgesehen sein, dass mittels des Überwachungsmittels anhand eines ermittelten Druckes in wenigstens einem Abschnitt des extrakorporalen Blutkreislaufes eine Stelle im extrakorporalen Blutkreislauf mit einem erhöhten Flusswiderstand ermittelbar ist bzw. bei Vorliegen eines erhöhten Flusswiderstandes ermittelt wird.

Weitere Einzelheiten und Vorteile der Erfindung sollen nun anhand eines in der Zeichnung dargestellten Ausführungsbeispiels näher erläutert werden.

Es zeigen:

Figur 1: eine schematische Darstellung des extrakorporalen Kreislaufs mit Zentrifugalpumpe, Dialysator, Flusssensor und Sicherheitsklemmen;

Figur 2: ein Diagramm betreffend den Durchfluss in Abhängigkeit der Drehzahl bei unterschiedlichen Flusswiderständen und konstanter Viskosität;

Figur 3: ein Diagramm betreffend den Durchfluss in Abhängigkeit der Drehzahl bei unterschiedlicher Viskosität und konstantem Flusswiderstand;

Figur 4: ein Ablaufdiagramm einer reversiblen Drehzahländerung zur Flusswiderstandsbestimmung; und

Figur 5: ein weiteres Ablaufdiagramm für die Inbetriebnahme der erfindungsgemäßen Blutbehandlungsvorrichtung.

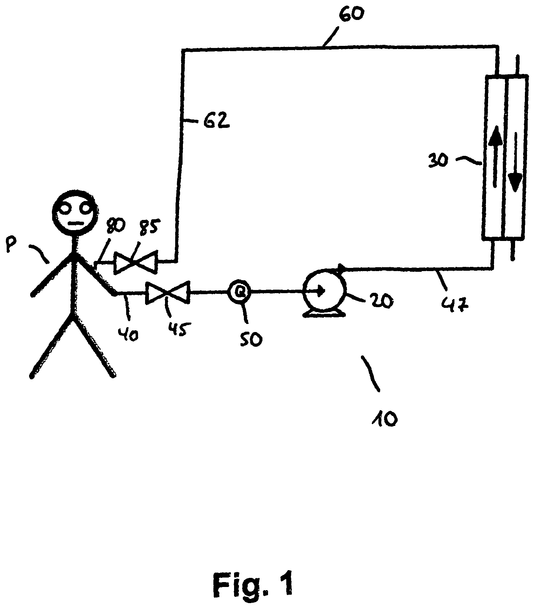

Figur 1 zeigt in schematischer Darstellung einen extrakorporalen Blutkreislauf 10 einer erfindungsgemäßen Blutbehandlungsvorrichtung mit einer Zentrifugalpumpe 20, Dialysator 30, Flusssensor 50 und Sicherheitsklemmen 45 und 85.

Der extrakorporale Blutkreislauf 10 ist dabei Bestandteil einer Blutbehandlungsvorrichtung und wird durch die Zentrifugalpumpe 20 angetrieben. Stromabwärts der Zentrifugalpumpe 20 ist ein Dialysator 30 angeordnet, wobei es sich bei dem Dialysator 30 um einen Hohlfasermembrandialysator 30 handeln kann bzw. vorzugsweise handelt.

Der Patient P wird über einen nicht näher dargestellten Katheter oder Nadeln an den extrakorporalen Blutkreislauf 10 angeschlossen, wobei der arterielle Anschluss 40 mit einer Klemme 45 versehen ist, so dass der arterielle Anschluss 40 abgeklemmt werden kann. Zwischen der Klemme 45 und der Zentrifugalpumpe 20 befindet sich ein Flusssensor 50. Der arterielle Teil des extrakorporalen Blutkreislaufs 10 ist mit dem Bezugszeichen 47 bezeichnet.

Der venöse Teil 60 des extrakorporalen Blutkreislaufs 10 umfasst die Rückführleitung 62, die zum venösen Anschluss 80 führt, mit dem dem Patienten P das behandelte Blut zurückgegeben wird. Vor dem venösen Anschluss 80 ist eine Klemme 85 vorgesehen, mittels dessen dieser Anschluss abgeklemmt werden kann.

Die vorliegende Erfindung basiert insbesondere auf der Erkenntnis, dass der Differenzdruck, der durch eine Zentrifugalpumpe wie durch die Zentrifugalpumpe 20 erzeugt wird, näherungsweise proportional zum Quadrat der Drehzahl der Zentrifugalpumpe ist:

$$\Delta p \sim n^2$$

(Gleichung 1)

Weiterhin ist der Durchfluss bei konstanter Viskosität und konstantem Flusswiderstand näherungsweise proportional zur Quadratwurzel des Differenzdruckes:

$$Q \sim \sqrt{\Delta p}$$

(Gleichung 2)

Daraus folgt, dass der Durchfluss bei konstantem Widerstand und konstanter Viskosität proportional zur Drehzahl der Zentrifugalpumpe ist:

$$Q \sim n$$

(Gleichung 3)

mit

p = Druck,
Q = Durchfluss,
n = Drehzahl.

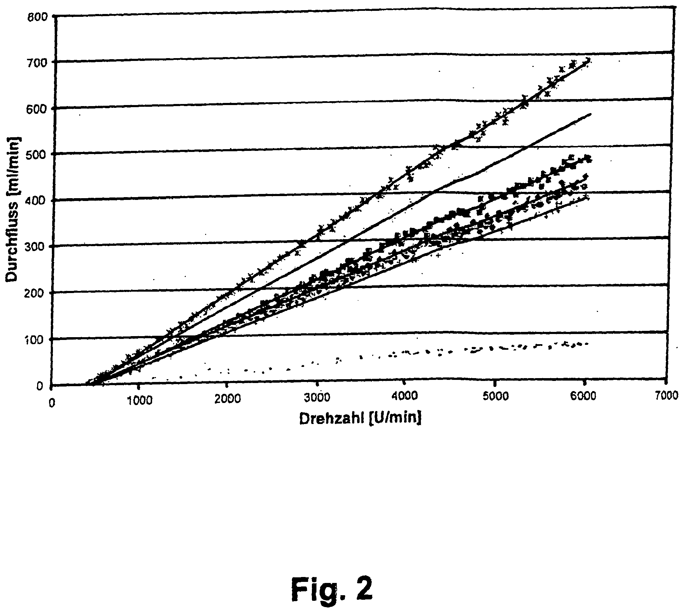

Die Steigung dieses Zusammenhangs ist abhängig vom Flusswiderstand des Kreislaufs, wie sich dies insbesondere aus Figur 2 ergibt, die ein Diagramm betreffend den Durchfluss in Abhängigkeit der Drehzahl bei unterschiedlichen Flusswiderständen und konstanter Viskosität zeigt.

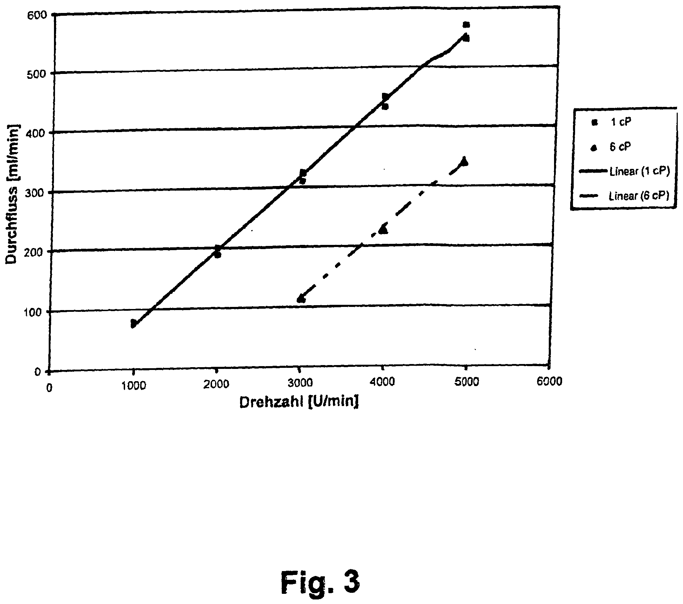

Dabei wirkt sich die Viskosität der Flüssigkeit als Offset aus, wie sich dies weiter aus Figur 3 ergibt. Dadurch kann man durch die Ermittlung des Flussanstiegs bei variabler Drehzahl ein Maß für den Flusswiderstand des Kreislaufs ermitteln.

Während einer Dialysebehandlung treten sowohl Änderungen der Viskosität, etwa durch Ultrafiltration etc., als auch Änderungen des Flusswiderstands durch Kinking, Clotting, Nadelansaugen etc. auf. Beide Änderungen haben einen direkten Einfluss auf den resultierenden Durchfluss.

Da der Durchfluss ein wichtiger Parameter der Dialysebehandlung ist, sollte dieser im Mittel dem voreingestellten Wert entsprechen. Dies wird durch eine Regelung mittels Flusssensor erreicht. Änderungen in Viskosität und Flusswiderstand führen jedoch dazu, dass die Regelung die Drehzahl der Zentrifugalpumpe verändern muss. Während des Regelvorgangs werden die dadurch verursachten Flussänderungen genutzt, um die Steigung dQ/dn zu bestimmen, die ein Maß für den Flusswiderstand ist.

Dadurch ist bekannt, ob die Änderung durch die Viskosität der Flüssigkeit oder den Flusswiderstand des Kreislaufs hervorgerufen wurde. Die Druckmessung im extrakorporalen Kreislauf ist hierzu nicht in der Lage, da sowohl Widerstands- als auch Viskositätsänderungen zu einer Änderung des Drucks führen.

Zudem ist stets ein aktuelles Maß für den Flusswiderstand bekannt. Dieses Maß kann genutzt werden, um einerseits Gegenmaßnahmen einzuleiten und andererseits einen leicht verständlichen, nicht-technischen Indikator für den Flusswiderstand beispielsweise auf einem User-Interface, z. B. einem Display der Blutbehandlungsvorrichtung darzustellen, z. B. in Form einer Batteriefüllstandsanzeige oder ähnlichem.

Diese Ermittlung des Differenzdruckes kann folgendermaßen beispielsweise bei der in Figur 1 gezeigten Blutbehandlungsvorrichtung eingesetzt werden:

So kann beispielsweise vor jeder Behandlung, insbesondere einer Dialysebehandlung ein Test der verwendeten Einwegartikel stattfinden. Vor jeder Behandlung werden die extrakorporalen Kreisläufe von Dialysemaschinen in der Regel mit Kochsalzlösungen gefüllt. Bei kurz geschlossenen Patientenleitungen wird dabei die Drehzahl variiert und der resultierende Fluss gemessen. Das Maß für den Flusswiderstand dQ/dn muss dabei innerhalb eines bestimmten Bereichs liegen, wenn der Kreislauf fehlerfrei ist. Andernfalls wird das Vorliegen von fehlerhaften Komponenten detektiert. Mit diesem Test kann zugleich der Flusssensor des extrakorporalen Blutkreislaufs der Dialysemaschine funktional getestet werden.

Ferner ist es möglich, eine kontinuierliche Überwachung des Flusswiderstands während der Behandlung durchzuführen. Dabei wird der Durchfluss mittels Regelung im Mittel auf einen voreingestellten Wert eingeregelt. Bei der Ausregelung von Störgrößen wie Flusswiderstand oder Viskosität wird neben der Drehzahländerung auch die Flussänderung beobachtet, um daraus das Maß für den Flusswiderstand dQ/dn herzuleiten.

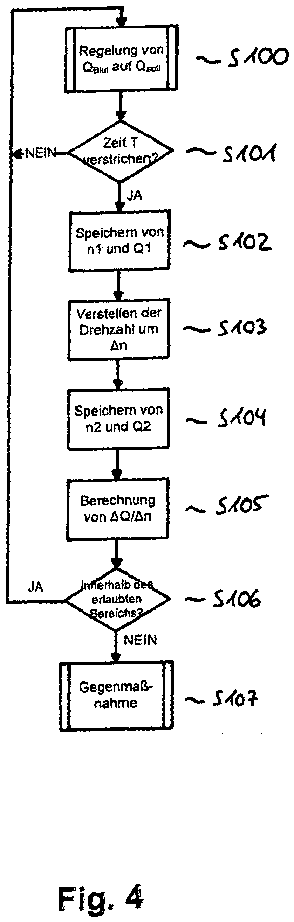

Verhältnismäßig schnelle Vorgänge wie Kinking, Clotting oder das Ansaugen eines Katheters bzw. der arteriellen Nadel führen dazu, dass der Durchfluss zunächst absinkt und anschließend wieder ausgeregelt wird. Langsame Vorgänge, wie die Änderung der Viskosität durch Ultrafiltration werden kontinuierlich ausgeregelt. Als eine weitere Sicherheitsmaßnahme kann eine reversible Drehzahländerung durchgeführt werden, die entweder zeitlich gesteuert oder bei Überschreitung einer bestimmten Drehzahl beim bestimmten Fluss hervorgerufen wird. Ein Beispiel für diesen Vorgang ist in Figur 4 dargestellt. Figur 4 zeigt dabei ein Ablaufdiagramm einer reversiblen Drehzahländerung zur Flusswiderstandsbestimmung.

In Schritt S100 erfolgt eine Regelung von $Q_{Blut}$ auf $Q_{Soll}$.

In Schritt S101 wird überprüft, ob eine bestimmte Zeitspanne T verstrichen ist. Sofern dies noch nicht der Fall ist, wird zu Schritt S100 zurückgekehrt. Sofern jedoch eine bestimmte Zeitspanne T bereits verstrichen ist, wird mit Schritt S102 fortgefahren, der darin besteht, die Werte für n1 und Q1 einzuspeichern.

Sodann erfolgt in Schritt S103 ein Verstellen der Drehzahl um Δn.

Sodann erfolgt in Schritt S104 das Einspeichern von n2 und Q2. In Schritt S105 kann sodann ΔQ/Δn berechnet werden.

In Schritt S106 wird sodann überprüft, ob der berechnete Wert ΔQ/Δn sich innerhalb eines erlaubten Bereichs befindet. Ist dies der Fall, wird zurück in Schritt S100 gesprungen. Sollte dies nicht der Fall sein, erfolgt in Schritt S107 eine entsprechende Gegenmaßnahme oder entsprechende Gegenmaßnahmen.

Die Detektion eines zu stark gestiegenen Flusswiderstands kann genutzt werden, um eine automatische Blutrückgabe einzuleiten, was beispielsweise eine Gegenmaßnahme bzw. Bestandteil des Schrittes S107 sein kann. Eine weitere Gegenmaßnahme gemäß Schritt S107 kann auch darin bestehen, die Behandlung im Sinne der Patientensicherheit zu unterbrechen oder einen anders gearteten sicheren Zustand einzuleiten.

Darüber hinaus ist es möglich, eine leicht verständliche Darstellung des Flusswiderstandes für Bediener und Patienten der Blutbehandlungsvorrichtung zu ermöglichen.

Da der Wert dQ/dn ein Maß für den Flusswiderstand des Blutkreislaufs bzw. extrakorporalen Blutkreislaufs 10 ist, kann dieser Wert auch genutzt werden, um eine leicht verständliche und nicht-technische Darstellung des Flusswiderstands für den Bediener des Gerätes zur Verfügung zu stellen (z. B. im Stil einer Batteriefüllstandsanzeige).

Dabei kann beispielsweise der Wert von dQ/dn, der beim initialen Test des Einwegartikels genutzt werden, um den Wert zu normieren. So würden 100 % dem initialen Wert der Steigung entsprechen, während 0 % ein Wert ist, über den das Fördern von Blut durch den extrakoporalen Kreislauf 10 nicht mehr ohne stark erhöhte Blutschädigung durchgeführt werden kann.

Darüber hinaus ist es möglich, eine Ortung der Flusswiderstandserhöhung, also der Stelle, an der die Erhöhung des Flusswiderstands hervorgerufen wird, zu detektieren.

Ein zusätzlicher Drucksensor kann beispielsweise im extrakorporalen Blutkreislauf 10 angebracht werden, etwa zwischen der Zentrifugalpumpe 20 und einem Dialysator 30. Hierdurch wird es ermöglicht, einen erhöhten Flusswiderstand zu orten. Fällt der gemessene Druck ab, so befindet sich der erhöhte Flusswiderstand zwischen Patient P und Pumpe 20. Steigt der gemessene Druck an, so befindet sich der erhöhte Flusswiderstand hinter der Pumpe 20, z. B. im Dialysator 30. Durch die Ortung wird es ermöglicht, gezielt Gegenmaßnahmen einleiten zu können.

Bei einem angesaugten Katheter bzw. Nadel wird beispielsweise die Drehzahl der Blutpumpe 20 und damit der Saugdruck stark gedrosselt. Da die Pumpe 20 nicht okkludierend ist, verbleibt der hohe Unterdruck nicht zwischen Gefäßwand und Katheter bzw. Nadel, sondern wird abgebaut. Anschließend kann Druck oder zeitgesteuert die Drehzahl der Zentrifugalpumpe 20 wieder gesteigert und die Behandlung fortgesetzt werden. Bleibt die Engstelle bestehen, beispielsweise durch Kinking des Patientenschlauches, muss der sichere Zustand eingeleitet werden, also ein Abbruch der Behandlung vorgenommen und ein Alarm ausgegeben werden.

Ferner ist es möglich, die Viskosität zu bestimmen. Wie sich dies aus Figur 1 und 2 ergibt, ist es möglich, die Viskosität der Flüssigkeit aus der Drehzahl der Pumpe 20 und dem Durchfluss des Systems zu bestimmen. Beschreibt man den Zusammenhang von Drehzahl und Durchfluss näherungsweise mit

$$Q \approx a * n + b$$

(Gleichung 4)

so entspricht a der Steigung dQ/dn bzw. einem vom Flusswiderstand abhängigen Parameter und b dem Offset bzw. einem von der Viskosität abhängigen Parameter. Hat man wie beschrieben den Flusswiderstands-Parameter a bestimmt, so berechnet sich b aus aktuellem / gemittelten Fluss und aktueller / gemittelter Drehzahl zu:

$$b_{aktuell} \approx Q_{aktuell} - \frac{\Delta Q}{\Delta n} n_{aktuell}$$

(Gleichung 5)

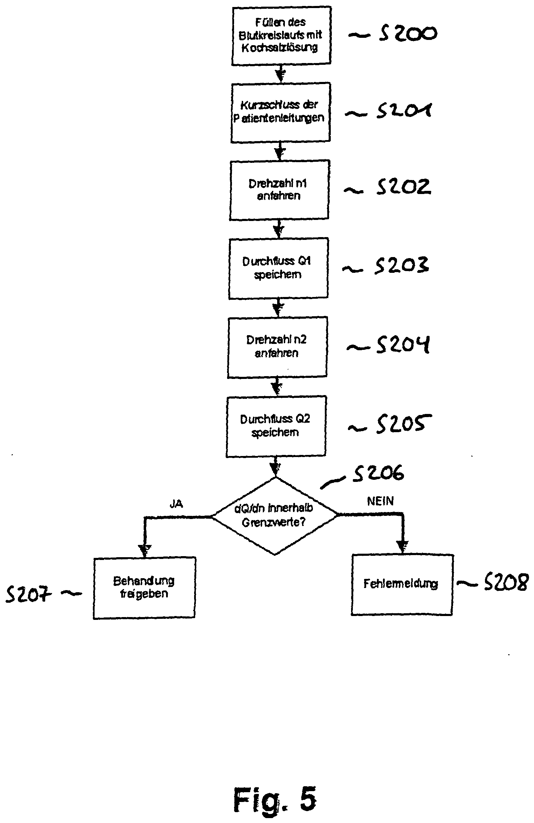

Figur 5 zeigt ein Ablaufdiagramm betreffend die initialen Tests bei der Inbetriebnahme des extrakorporalen Blutkreislaufs 10 gemäß einem Ausführungsbeispiel der vorliegenden Erfindung. Dabei wird im Schritt S200 das Füllen des extrakorporalen Blutkreislaufs 10 mit einer Kochsalzlösung durchgeführt. Sodann folgt in Schritt S201 ein Kurzschluss der Patientenleitungen. In Schritt S202 wird sodann die Drehzahl der Zentrifugalpumpe 20 gemäß Figur 1 mit einer Drehzahl n1 angefahren. In Schritt S203 wird sodann der Durchfluss Q1 gespeichert.

In Schritt S204 wird sodann die Drehzahl auf n2 erhöht und der extrakoporale Blutkreislauf 10 damit betrieben.

In Schritt S205 wird ferner der Durchfluss Q2 gespeichert.

In Schritt S206 wird daraufhin der Wert dQ/dn errechnet und überprüft, ob dieser innerhalb vorgegebener Grenzwerte liegt. Sollte dies der Fall sein, wird in Schritt S207 die Behandlung freigegeben und sofern dies nicht der Fall sein sollte, in Schritt S208 dementsprechend eine Fehlermeldung ausgegeben.

## Patentansprüche

1. Blutbehandlungsvorrichtung mit wenigstens einem extrakorporalen Blutkreislauf (10) mit einem Dialysator (30) sowie mit wenigstens einem Zentrifugalpumpmittel (20), wobei ferner ein Überwachungsmittel vorgesehen ist, mittels dessen der Flusswiderstand des extrakorporalen Kreislaufs (10) bei Inbetriebnahme des extrakorporalen Kreislaufs (10) in einem initialen Test bei kurzgeschlossenen Patientenleitungen ermittelt wird, wobei der Flusswiderstand zumindest teilweise anhand des Flusses (Q) in wenigstens einem Abschnitt des extrakorporalen Blutkreislaufs und der Drehzahl (n) des Zentrifugalpumpmittels ermittelt wird und wobei der extrakorporale Blutkreislauf (10) zumindest teilweise durch eine disposable Kassette ausgebildet ist, wobei mittels des Überwachungsmittels in einem ersten Schritt der Fluss (Q1) in dem wenigstens einen Abschnitt des extrakorporalen Blutkreislaufs (10) bei einer ersten Drehzahl (n1) ermittelt wird und in einem zweiten Schritt der Fluss (Q2) in dem wenigstens einen Abschnitt des extrakorporalen Blutkreislaufs (10) bei einer zweiten Drehzahl (n2) ermittelt wird, wobei anhand dieser Werte (Q1, Q2; n1, n2) als Maß für den Flusswiderstand (dQ/dn) das Verhältnis der Differenzwerte errechnet wird, und wobei mittels des Überwachungsmittels ein fehlerhafter Zustand des extrakorporalen Blutkreislaufs (10) erkannt wird, wenn das Maß für den Flusswiderstand (dQ/dn) einen vordefinierten Bereich verlässt und/oder einen vordefinierte Schwellwert übersteigt oder unterschreitet, wobei die Behandlung freigegeben wird, wenn das Maß für den Flusswiderstand (dQ/dn) innerhalb vorgegebener Grenzwerte liegt, und, falls dies nicht der Fall ist, eine Fehlermeldung ausgegeben wird.

2. Blutbehandlungsvorrichtung nach Anspruch 1, **dadurch gekennzeichnet, dass** wenigstens ein Anzeigemittel vorgesehen ist, mittels dessen das Maß für den Flusswiderstand (dQ/dn) dargestellt und/oder angezeigt wird.

3. Blutbehandlungsvorrichtung nach einem der vorhergehenden Ansprüche, **dadurch gekennzeichnet, dass** im extrakorporalen Blutkreislauf (10) wenigstens ein Druckerfassungsmittel, insbesondere wenigstens ein Drucksensor, vorgesehen ist, mittels dessen der Druck in wenigstens einem Abschnitt des extrakorporalen Blutkreislaufs ermittelt wird.

4. Blutbehandlungsvorrichtung nach Anspruch 3, **dadurch gekennzeichnet, dass** mittels des Überwachungsmittels anhand des mittels des Druckerfassungsmittels ermittelten Druckes eine Stelle im extrakorporalen Blutkreislauf mit einem erhöhten Flusswiderstand ermittelt wird.

5. Blutbehandlungsvorrichtung nach einem der vorhergehenden Ansprüche, **dadurch gekennzeichnet, dass** die Kassette wenigstens einen Schlauchabschnitt und/oder wenigstens eine Tropfkammer und/oder wenigstens ein Ventil und/oder wenigstens einen Filter und/oder den Dialysator (30) aufweist.

6. Verfahren zur Inbetriebnahmeeiner Blutbehandlungsvorrichtung mit wenigstens einem extrakorporalen Blutkreislauf (10) mit einem Dialysator (30) sowie mit wenigstens einem Zentrifugalpumpmittel (20), wobei ferner ein Überwachungsmittel vorgesehen ist, mittels dessen der Flusswiderstand des extrakorporalen Kreislaufs (10) bei Inbetriebnahme des extrakorporalen Kreislaufs (10) in einem initialen Test bei kurzgeschlossenen Patientenleitungen ermittelt wird, wobei der Flusswiderstand zumindest teilweise anhand des Flusses (Q) in wenigstens einem Abschnitt des extrakorporalen Blutkreislaufs und der Drehzahl (n) des Zentrifugalpumpmittels ermittelt wird, wobei der extrakorporale Blutkreislauf (10) zumindest teilweise durch eine disposable Kassette ausgebildet ist, wobei mittels des Überwachungsmittels in einem ersten Schritt der Fluss (Q1) in dem wenigstens einen Abschnitt des extrakorporalen Blutkreislaufs (10) bei einer ersten Drehzahl (n1) ermittelt wird und in einem zweiten Schritt der Fluss (Q2) in dem wenigstens einen Abschnitt des extrakorporalen Blutkreislaufs (10) bei einer zweiten Drehzahl (n2) ermittelt wird, wobei anhand dieser Werte (Q1, Q2; n1, n2) als Maß für den Flusswiderstand (dQ/dn) das Verhältnis der Differenzwerte errechnet wird, wobei mittels des Überwachungsmittels ein fehlerhafter Zustand des extrakorporalen Blutkreislaufs (10) erkannt wird, wenn das Maß für den Flusswiderstand (dQ/dn) einen vordefinierten Bereich verlässt und/oder einen vordefinierte Schwellwert übersteigt oder unterschreitet, wobei die Behandlung freigegeben wird, wenn das Maß für den Flusswiderstand (dQ/dn) innerhalb vorgegebener Grenzwerte liegt, und, falls dies nicht der Fall ist, eine Fehlermeldung ausgegeben wird.

**7.** Verfahren nach Anspruch 6, **dadurch gekennzeichnet, dass** mittels des Überwachungsmittels anhand eines ermittelten Druckes in wenigstens einem Abschnitt des extrakorporalen Blutkreislaufes eine Stelle im extrakorporalen Blutkreislauf mit einem erhöhten Flusswiderstand ermittelbar ist oder bei Vorliegen eines erhöhten Flusswiderstandes ermittelt wird.

**8.** Verfahren nach einem der Ansprüche 6 bis 7 zum Betrieb einer Blutbehandlungsvorrichtung gemäß einem der Ansprüche 1 bis 5.

**Claims**

**1.** A blood treatment device with at least one extracorporeal blood circuit (10) with a dialyzer (30) and with at least one centrifugal pump means (20), wherein furthermore a monitoring means is provided, by means of which the flow resistance of the extracorporeal circuit (10) at start-up of the extracorporeal circuit (10) is determined in an initial test with short-circuited patient lines, wherein the flow resistance is at least partly determined with reference to the flow (Q) in at least one portion of the extracorporeal blood circuit and the speed (n) of the centrifugal pump means, and wherein the extracorporeal blood circuit (10) is at least partly formed by a disposable cassette, wherein in a first step the flow (Q1) in the at least one portion of the extracorporeal blood circuit (10) is determined at a first speed (n1) by means of the monitoring means and in a second step the flow (Q2) in the at least one portion of the extracorporeal blood circuit (10) is determined at a second speed (n2), wherein with reference to these values (Q1, Q2; n1, n2) the ratio of the differential values is calculated as a measure for the flow resistance (dQ/dn), and wherein by means of the monitoring means a faulty condition of the extracorporeal blood circuit (10) is detected when the measure for the flow resistance (dQ/dn) leaves a predefined range and/or exceeds or falls below a predefined threshold value, wherein the treatment is enabled when the measure for the flow resistance (dQ/dn) lies within specified limit values, and, if this is not the case, an error message is issued.

**2.** The blood treatment device according to claim 1, **characterized in that** at least one display means is provided, by means of which the measure for the flow resistance (dQ/dn) is represented and/or displayed.

**3.** The blood treatment device according to any of the preceding claims, **characterized in that** in the extracorporeal blood circuit (10) at least one pressure detection means, in particular at least one pressure sensor, is provided, by means of which the pressure in at least one portion of the extracorporeal blood circuit is determined.

**4.** The blood treatment device according to claim 3, **characterized in that** by means of the monitoring means a point in the extracorporeal blood circuit with an increased flow resistance is determined with reference to the pressure determined by means of the pressure detection means.

**5.** The blood treatment device according to any of the preceding claims, **characterized in that** the cassette includes at least one hose portion and/or at least one drip chamber and/or at least one valve and/or at least one filter and/or the dialyzer (30).

**6.** A method for operating a blood treatment device comprising at least one extracorporeal blood circuit (10) with a dialyzer (30) and with at least one centrifugal pump means (20), wherein furthermore a monitoring means is provided, by means of which the flow resistance of the extracorporeal circuit (10) at startup of the extracorporeal circuit (10) is determined in an initial test with short-circuited patient lines, wherein the flow resistance is at least partly determined with reference to the flow (Q) in at least one portion of the extracorporeal blood circuit and the speed (n) of the centrifugal pump means, wherein the extracorporeal blood circuit (10) is at least partly formed by a disposable cassette, wherein in a first step the flow (Q1) in the at least one portion of the extracorporeal blood circuit (10) is determined at a first speed (n1) by means of the monitoring means and in a second step the flow (Q2) in the at least one portion of the extracorporeal blood circuit (10) is determined at a second speed (n2), wherein with reference to these values (Q1, Q2; n1, n2) the ratio of the differential values is calculated as a measure for the flow resistance (dQ/dn), wherein by means of the monitoring means a faulty condition of the extracorporeal blood circuit (10) is detected, when the measure for the flow resistance (dQ/dn) leaves a predefined range and/or exceeds or falls below a predefined threshold value, wherein the treatment is enabled when the measure for the flow resistance (dQ/dn) lies within specified limit values, and, if this is not the case, an error message is issued.

**7.** The method according to claim 6, **characterized in that** by means of the monitoring means a point in the extracorporeal blood circuit with an increased flow resistance can be determined with reference to a determined pressure in at least one portion of the extracorporeal blood circuit or is determined in the presence of an increased flow resistance.

8. The method according to any of claims 6 to 7 for operating a blood treatment device according to any of claims 1 to 5.

**Revendications**

1. Dispositif de traitement de sang comportant au moins un circuit sanguin extracorporel (10) avec un dialyseur (30) et avec au moins un moyen de pompe centrifuge (20), dans lequel en outre un moyen de surveillance est prévu, au moyen duquel la résistance d'écoulement du circuit sanguin extracorporel (10) lors de la mise en service du circuit extracorporel (10) est déterminée dans un test initial avec des lignes de patient court-circuitées, dans lequel la résistance d'écoulement au moins partiellement est déterminée sur la base de l'écoulement (Q) dans au moins un tronçon du circuit sanguin extracorporel et la vitesse de rotation (n) du moyen de pompe centrifuge, et dans lequel le circuit sanguin extracorporel (10) est formé au moins partiellement par une cassette jetable, dans lequel dans une première étape l'écoulement (Q1) dans le au moins un tronçon du circuit sanguin extracorporel (10) à une première vitesse de rotation (n1) est déterminé au moyen du moyen de surveillance, et dans une deuxième étape l'écoulement (Q2) dans le au moins un tronçon du circuit sanguin extracorporel (10) est déterminé à une deuxième vitesse de rotation (n2), dans lequel sur la base de ces valeurs (Q1, Q2; n1, n2) le rapport des valeurs différentielles est calculé comme mesure de la résistance d'écoulement (dQ/dn), et dans lequel au moyen du moyen de surveillance un état défectueux du circuit sanguin extracorporel (10) est détecté, quand la mesure de la résistance d'écoulement (dQ/dn) quitte une plage prédéfinie et/ou dépasse ou tombe au-dessous une valeur seuil prédéfinie, dans lequel le traitement est activé quand la mesure de la résistance d'écoulement (dQ/dn) se trouve dans des valeurs limites spécifiées, et, si cela n'est pas le cas, un message d'erreur est émis.

2. Dispositif de traitement de sang selon la revendication 1, **caractérisé en ce qu'**au moins un moyen d'affichage est prévu, au moyen duquel la mesure de la résistance d'écoulement (dQ/dn) est représentée et/ou affichée.

3. Dispositif de traitement de sang selon l'une quelconque des revendications précédentes, **caractérisé en ce que** dans le circuit sanguin extracorporel (10) au moins un moyen de détection de pression, en particulier au moins un capteur de pression, est prévu, au moyen duquel la pression dans au moins un tronçon du circuit sanguin extracorporel est déterminée.

4. Dispositif de traitement de sang selon la revendication 3, **caractérisé en ce que** sur la base de la pression déterminée au moyen du moyen de détection de pression un endroit dans le circuit sanguin extracorporel avec une résistance d'écoulement élevée est déterminé au moyen du moyen de surveillance.

5. Dispositif de traitement de sang selon l'une quelconque des revendications précédentes, **caractérisé en ce que** la cassette comprend au moins un tronçon de flexible et/ou au moins une chambre compte-gouttes et/ou au moins une soupape et/ou au moins un filtre et/ou le dialyseur (30).

6. Procédé de mise en service d'un dispositif de traitement de sang comportant au moins un circuit sanguin extracorporel (10) avec un dialyseur (30) et avec au moins un moyen de pompe centrifuge (20), dans lequel en outre un moyen de surveillance est prévu, au moyen duquel la résistance d'écoulement du circuit sanguin extracorporel (10) lors de la mise en service du circuit extracorporel (10) est déterminée dans un test initial avec des lignes de patient court-circuitées, dans lequel la résistance d'écoulement au moins partiellement est déterminée sur la base de l'écoulement (Q) dans au moins un tronçon du circuit sanguin extracorporel et la vitesse de rotation (n) du moyen de pompe centrifuge, et dans lequel le circuit sanguin extracorporel (10) est formé au moins partiellement par une cassette jetable, dans lequel dans une première étape l'écoulement (Q1) dans le au moins un tronçon du circuit sanguin extracorporel (10) à une première vitesse de rotation (n1) est déterminé au moyen du moyen de surveillance, et dans une deuxième étape l'écoulement (Q2) dans le au moins un tronçon du circuit sanguin extracorporel (10) est déterminé à une deuxième vitesse de rotation (n2), dans lequel sur la base de ces valeurs (Q1, Q2; n1, n2) le rapport des valeurs différentielles est calculé comme mesure de la résistance d'écoulement (dQ/dn), dans lequel au moyen du moyen de surveillance un état défectueux du circuit sanguin extracorporel (10) est détecté, quand la mesure de la résistance d'écoulement (dQ/dn) quitte une plage prédéfinie et/ou dépasse ou tombe au-dessous une valeur seuil prédéfinie, dans lequel le traitement est activé quand la mesure de la résistance d'écoulement (dQ/dn) se trouve dans des valeurs limites spécifiées, et, si cela n'est pas le cas, un message d'erreur est émis.

**7.** Procédé selon la revendication 6, **caractérisé en ce que** sur la base d'une pression déterminée dans au moyen un tronçon du circuit sanguin extracorporel un endroit dans le circuit sanguin extracorporel avec une résistance d'écoulement élevée peut être déterminé au moyen du moyen de surveillance ou est déterminé en présence d'une résistance d'écoulement élevée.

**8.** Procédé selon l'une quelconque des revendications 6 à 7 pour faire fonctionner un dispositif de traitement de sang selon l'une quelconque des revendications 1 à 5.

**Fig. 1**

**Fig. 2**

**Fig. 3**

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102007007198 A1
- DE 102009024465 A1
- DE 102009018664 A1